# EUROPEAN PATENT APPLICATION

(11) **EP 4 678 740 A1**
(43) Date of publication of application: **14.01.2026**
(21) Application number: 24767119.1
(22) Date of filing: 05.03.2024
(51) Int. Cl.: C12N 5/0797, A61K 35/30, A61L 27/38, A61L 27/40, A61L 27/50, A61P 27/02, C12N 5/10, C12N 5/0735, C07K 14/78, C12N 5/071

(54) **TISSUE CONTAINING NEURAL RETINA PROGENITOR CELLS, PRODUCTION METHOD THEREOF, AND COMPOSITION FOR TREATING RETINAL DISEASES COMPRISING SAID TISSUE**

(30) Priority: 06.03.2023 JP 2023033455
(71) Applicant: VCCT Inc., Kobe-shi, Hyogo 650-0047 (JP); Kobe City Hospital Organization, Kobe-shi, Hyogo 650-0047 (JP)
(72) Inventor: MASUDA, Tomohiro, Kobe-shi, Hyogo 650-0047 (JP); NISHIDA, Mitsuhiro, Kobe-shi, Hyogo 650-0047 (JP); MANDAI, Michiko, Kobe-shi, Hyogo 650-0047 (JP)
(74) Representative: Jones, Nicholas Andrew
(86) International application number: PCT/JP2024/008169
(87) International publication number: WO 2024/185751

(57) **Abstract**

As a technique for obtaining a neural retinal sheet with improved uniformity of the thickness of the neural retinal progenitor cell layer, provided is a method of producing a tissue comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor, wherein the surface of the region A is coated with a cell-adhesive material, and has a contact angle within a predetermined range before being coated with the cell-adhesive material.

## Description

### Technical Field

The present disclosure relates to a tissue containing neural retinal progenitor cells, a production method thereof, and a composition for treating retinal diseases containing the tissue. More specifically, it relates to a method for producing a sheet-like tissue containing neural retinal progenitor cells by culturing pluripotent stem cells in an adhesive state in the presence of a BMP signaling inhibitor, and then in the presence of a BMP signaling activator, and the like.

### Background Art

In the treatment of retinal diseases, attempts have been made to restore visual function by transplanting neural retinal cells differentiated from embryonic stem cells (ES cells) and induced pluripotent stem cells (iPS cells), a neural retinal sheet excised from a retinal organoid differentiated from ES/iPS cells, or the like.

Patent Literature 1 discloses a "method for producing a retinal tissue, the method comprising inducing differentiation of pluripotent stem cells into a retinal tissue in a region A on a culture substrate having, on its surface, a region A having cell adhesiveness and a region B adjacent to at least a part of the region A and having less cell adhesiveness than the region A," as a technique for obtaining a neural retinal sheet for transplantation. According to this technique (hereinafter referred to as "planar retinal organoid differentiation method"), it is possible to obtain a circular sheet-like retinal tissue with a maximum diameter of about 10 mm, by culturing pluripotent stem cells in an adhesive state in the presence of a BMP signaling inhibitor, and then in the presence of a BMP signaling activator, in the region A. In the sheet-like retinal tissue, when the substrate surface is defined as the x-y plane, a neural retinal progenitor cell layer is formed along the z-axis direction, and the cells constituting the retinal tissue autonomously arrange themselves with the substrate surface side in the z-axis direction as the basal side (ganglion cell side) and the opposite side as the apical side (photoreceptor cell side). The neural retinal sheet for transplantation is obtained by excising this neural retinal progenitor cell layer.

### Citation List

### Patent Literature

Patent Literature 1: WO2023/003025

### Summary of Invention

### Technical Problem

In order for the neural retinal sheet for transplantation to have a therapeutic effect of restoring visual function, it is preferable that the neural retinal progenitor cell layer be formed with a uniform thickness. A primary object of the present disclosure is to provide a technique for obtaining a neural retinal sheet with improved uniformity of the thickness of the neural retinal progenitor cell layer.

### Solution to Problem

In order to solve the above problems, the present disclosure provides the following [1] to [28].
[1] A method for producing a tissue comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor,
   wherein the surface of the region A is coated with a cell-adhesive material, and has a contact angle within a predetermined range before being coated with the cell-adhesive material.
[2] The production method according to [1], wherein the surface of the region A has a contact angle of 40 to 80 degrees with a cell-adhesive material solution when coated with the cell-adhesive material.
[3] The production method according to [1] or [2], wherein the surface of the region A has a contact angle of 54 to 77 degrees with water when coated with the cell-adhesive material.
[4] The production method according to any one of [1] to [3], further comprising a step of adding dropwise the cell-adhesive material solution onto a partial region of the surface of the culture substrate to coat it with the cell-adhesive material and form the region A, and defining, as the region B, a region of the surface of the culture substrate that is not coated with the cell-adhesive material solution.
[5] The production method according to any one of [1] to [3], further comprising:
   a step of forming a low cell-adhesion layer on a surface of the culture substrate;
   a step of removing a portion of the low cell-adhesion layer on the surface of the culture substrate to expose the surface of the culture substrate; and
   a step of adding dropwise the cell-adhesive material solution onto the exposed region of the surface of the culture substrate to coat it with the cell-adhesive material and form the region A, and defining, as the region B, a region of the surface of the culture substrate where the low cell-adhesion layer has been maintained.
[6] The production method according to any one of [1] to [5], wherein the cell-adhesive material is one or more selected from the group consisting of positively charged polymers, laminin, collagens, tenascin, fibrillin, fibronectin, vitronectin, elastin, entactin, proteoglycans, glycosaminoglycans, vitronectin derivatives, and Matrigel.
[7] The production method according to any one of [1] to [6], wherein the cell-adhesive material is laminin.
[8] The production method according to any one of [1] to [7], wherein the cell-adhesive material is a human laminin 511-E8 fragment.
[9] The production method according to any one of [1] to [8], wherein the cell-adhesive material solution is a 7 ng/µl phosphate buffer solution (PBS) of human laminin 511-E8 fragment.
[10] The production method according to any one of [1] to [9], wherein the culture in an adhesive state comprises:
   a step 1 of culturing the pluripotent stem cells in a medium comprising a BMP signaling inhibitor, as well as serum and/or a serum substitute; a step 2, following the step 1, of culturing the cells obtained in the step 1 in a medium comprising a BMP signaling activator, as well as serum and/or a serum substitute; and a step 3, following the step 2, of culturing the cells obtained in the step 2 in a medium free of both BMP signaling inhibitor and BMP signaling activator, and comprising serum and optionally a serum substitute.
[11] The production method according to [10], which does not comprise any additional steps between the step 2 and the step 3.
[12] A method for producing a tissue comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor, wherein the culture in an adhesive state comprises: a step 1 of culturing the pluripotent stem cells in a medium comprising a BMP signaling inhibitor, as well as serum and/or a serum substitute; a step 2, following the step 1, of culturing the cells obtained in the step 1 in a medium comprising a BMP signaling activator, as well as serum and/or a serum substitute; and a step 3, following the step 2, of culturing the cells obtained in the step 2 in a medium free of both BMP signaling inhibitor and BMP signaling activator, and comprising serum and optionally a serum substitute.
[13] The production method according to [12], which does not comprise any additional steps between the step 2 and the step 3.
[14] The production method according to [12] or [13], wherein the surface of the region A is coated with a cell-adhesive material, and has a contact angle within a predetermined range before being coated with the cell-adhesive material.
[15] The production method according to [14], wherein the surface of the region A has a contact angle of 40 to 80 degrees with a cell-adhesive material solution when coated with the cell-adhesive material.
[16] The production method according to [14] or [15], wherein the surface of the region A has a contact angle of 54 to 77 degrees with water when coated with the cell-adhesive material.
[17] The production method according to any one of [12] to [16], further comprising a step of adding dropwise the cell-adhesive material solution onto a partial region of the surface of the culture substrate to coat it with the cell-adhesive material and form the region A, and defining, as the region B, a region of the surface of the culture substrate that is not coated with the cell-adhesive material solution.
[18] The production method according to any one of [12] to [16], further comprising:
   a step of forming a low cell-adhesion layer on a surface of the culture substrate;
   a step of removing a portion of the low cell-adhesion layer on the surface of the culture substrate to expose the surface of the culture substrate; and
   a step of adding dropwise the cell-adhesive material solution onto the exposed region of the surface of the culture substrate to coat it with the cell-adhesive material and form the region A, and defining, as the region B, a region of the surface of the culture substrate where the low cell-adhesion layer has been maintained.
[19] The production method according to any one of [14] to [18], wherein the cell-adhesive material is one or more selected from the group consisting of positively charged polymers, laminin, collagens, tenascin, fibrillin, fibronectin, vitronectin, elastin, entactin, proteoglycans, glycosaminoglycans, vitronectin derivatives, and Matrigel.
[20] The production method according to any one of [14] to [19], wherein the cell-adhesive material is laminin.
[21] The production method according to any one of [14] to [20], wherein the cell-adhesive material is a human laminin 511-E8 fragment.
[22] The production method according to any one of [14] to [21], wherein the cell-adhesive material solution is a 7 ng/µl phosphate buffer solution (PBS) of human laminin 511-E8 fragment.
[23] A tissue comprising neural retinal progenitor cells, obtained by the production method according to any one of [1] to [22].
[24] A composition for treating retinal diseases, comprising the tissue according to [23].
[25] A cell-based medicament for treating retinal diseases, comprising the tissue according to [23].
[26] A method for producing a neural retinal sheet for transplantation from a retinal organoid derived from pluripotent stem cells, the method comprising: a step of producing a tissue (retinal organoid) comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor, wherein the surface of the region A is coated with a cell-adhesive material, and has a contact angle within a predetermined range before being coated with the cell-adhesive material; and a step of excising a neural retinal progenitor cell layer from the tissue (retinal organoid) to obtain the neural retinal sheet for transplantation.
[27] A method for producing a composite sheet for retinal transplantation, the method comprising: a step of producing the neural retinal sheet for transplantation by the method of [26]; and a step of laminating the neural retinal sheet for transplantation onto a retinal pigment epithelial cell sheet using a flowable gel to produce the composite sheet for retinal transplantation.
[28] A method for treating retinal diseases, comprising a procedure of transplanting the tissue comprising neural retinal progenitor cells of [23], the composition for treating retinal diseases of [24], the cell-based medicament for treating retinal diseases of [25], the neural retinal sheet for transplantation of [26], or the composite sheet for retinal transplantation of [27] into the retina of a subject.

### [Definitions]

In the present disclosure, a "pluripotent stem cell" refers to an embryonic stem cell (ES cell) and a cell that potentially possesses pluripotency similar to that of ES cells. Examples of cells possessing pluripotency similar to that of ES cells include induced pluripotent stem cells (iPS cells), which are somatic cells that have been reprogrammed to acquire pluripotency and self-renewal capacity.

ES cells are pluripotent stem cells that are derived from early embryos, and various established ES cell lines can be utilized. Examples of ES cell lines that can be used include, but are not limited to, clinical-grade human ES cell lines provided by the Center for Human ES Cell Research of the Institute for Life and Medical Sciences, Kyoto University, and human ES cell lines (KhES-1, KhES-1_Crx: :Venus, and KhES-1_Rx::Venus) provided by RIKEN BRC.

As the iPS cells, not only iPS cells induced from the patient's own somatic cells but also various iPS cell lines can be utilized. Examples of iPS cell lines that can be used include, but are not limited to, various iPS stock cell lines provided by the CiRA Foundation, Kyoto University, and iPS cell lines derived from healthy donors provided by RIKEN BRC, such as KVs09 and CLs23.

The species of pluripotent stem cells is not limited and is appropriately selected according to the recipient. In the case of a retinal sheet for human transplantation, cells derived from primates, particularly those derived from monkeys or humans, are preferred, and human-derived pluripotent stem cells are more preferred.

The retinal tissue in a living body has a layered structure and contains photoreceptor cells, horizontal cells, bipolar cells, amacrine cells, retinal ganglion cells, retinal pigment epithelial cells, Mueller cells, and their progenitor cells.

In the present disclosure, the cells that make up the retina are referred to as "retinal cells" and each layer that makes up the retina is referred to as a "retinal layer." Retinal layers in a mature retinal tissue include a retinal pigment epithelial layer, photoreceptor layer, outer limiting membrane, outer nuclear layer, outer plexiform layer, inner nuclear layer, inner plexiform layer, ganglion cell layer, nerve fiber layer and inner limiting membrane. Retinal tissues in a developmental stage before becoming mature retinal tissues may include a neuroblastic layer.

The term "neural retinal progenitor cell" refers to a progenitor cell that can differentiate into one or more types of mature retinal cells selected from photoreceptor cell, horizontal cell, bipolar cell, amacrine cell, and retinal ganglion cell. Typically, a neural retinal progenitor cell does not differentiate into a retinal pigment epithelial cell, ciliary marginal zone cell, or the lens.

Examples of markers for neural retinal progenitor cells include RAX (RX), PAX6, VSX2, SIX3, and SIX6 (expressed in retinal progenitor cells); OTX2, CRX, PRDM1, RHO, RCVRN, NR2E3, NRL, RXR-gamma, MSI1, PROM1, OPN1SW, OPN1MW, and OPN1LW (expressed in photoreceptor cells); CALB1, ONECUT1, and ONECUT2 (expressed in horizontal cells); VSX2, ISL1, PKCα, and PCP2 (expressed in bipolar cells); CALB2 and PROX1 (expressed in amacrine cells); and TUBB3, POU4F2, and ISL1 (expressed in retinal ganglion cells).

The term "retinal pigment epithelium-like cell" refers to a cell that mimics a cell that forms the retinal pigment epithelium, which is a single layer of epithelial tissue. Examples of markers for retinal pigment epithelial cells include MITF, TTR, BEST1, RPE65, and MERTK.

The term "ciliary marginal zone-like cell" refers to a cell that mimics a cell that forms the intermediate region (marginal zone) between the ciliary body and the neural retina. Examples of markers for ciliary marginal zone cells include ZIC1, AQP1, and FUT4.

The term "lens" refers to a tissue that forms a colorless, transparent, convex-shaped lens. Examples of markers for the lens include CRYAB, CRYGA, CRYGB, and CRYGC.

A "retinal organoid" refers to a three-dimensional retinal tissue produced ex vivo. Accordingly, "retinal organoid derived from pluripotent stem cells" refers to a three-dimensional retinal tissue obtained by inducing the differentiation of pluripotent stem cells ex vivo. The retinal organoid is preferably a planar retinal organoid (see International Publication No. 2023/003025).

The term "differentiation-inducing factor" as used in the present disclosure encompasses at least a BMP signaling inhibitor and a BMP signaling activator.

The "BMP signaling inhibitor" is not limited as long as it is a substance that inhibits signaling by BMP. Examples of BMP signaling inhibitors include LDN-193189 (4-[6-(4-Piperazin-1-ylphenyl)pyrazolo[1,5-a]pyrimidin-3-yl]quinoline), Dorsomorphin (6-[4-[2-(1-Piperidinyl)ethoxy]phenyl]-3-(4-pyridinyl)-pyrazolo[1,5-a]pyrimidine), and DMH1 (4-(6-(4-isopropoxyphenyl)pyrazolo[1,5-a]pyrimidin-3-yl)quinoline).

The "BMP signaling activator" is not limited as long as it is a substance that activates signaling by BMP. Examples of BMP signaling activators include BMP2, BMP4, BMP7, BMP12 (GDF7) and fragments thereof, and anti-BMP receptor antibodies.

The term "serum" refers to the supernatant fraction obtained by removing blood cell components from coagulated blood. Examples of serum include human serum, fetal bovine serum (FBS), bovine serum, calf serum, goat serum, horse serum, porcine serum, sheep serum, rabbit serum, and rat serum; and it is preferably FBS, calf serum, or bovine serum, and more preferably FBS. The serum may or may not be inactivated.

The term "serum substitute" refers to a composition composed of known components that is used in place of serum. Examples of serum substitutes include albumin such as bovine serum albumin (BSA), transferrin, fatty acids, collagen precursors, trace elements, 2-mercaptoethanol, 1-thioglycerol, and their equivalents, and mixtures thereof.

### Advantageous Effects of Invention

The present disclosure provides a technique for obtaining a neural retinal sheet with improved uniformity of the thickness of the neural retinal progenitor cell layer.

### Brief Description of Drawings

[Figure 1] Figure 1 is a phase-contrast microscopic image on Day 37 of a planar retinal organoid prepared by the method of Test Example 1 using a dish A (contact angle of iMatrix-511 solution: 58.5 degrees).
[Figure 2] Figure 2 is a phase-contrast microscopic image on Day 37 of a planar retinal organoid prepared by the method of Reference Example 1 using a dish B (contact angle of iMatrix-511 solution: 30.0 degrees).
[Figure 3] Figure 3 is phase-contrast microscopic images on Day 38 of a planar retinal organoid prepared by the method of Reference Example 1 using dishes having different water contact angles: dish C (24 degrees), dish D (55 degrees), dish F (60 degrees), dish G (66 degrees), dish I (76 degrees), and dish J (90 degrees).
[Figure 4] Figure 4 is a phase-contrast microscopic image on Day 20 of a planar retinal organoid prepared by the method of Test Example 2.
[Figure 5] Figure 5 is a phase-contrast microscopic image on Day 20 of a planar retinal organoid prepared by the method of Reference Example 1.

### Description of Embodiments

### 1. Method for producing tissue comprising neural retinal progenitor cells

The method for producing a tissue comprising neural retinal progenitor cells according to the present disclosure is a method for producing a tissue comprising neural retinal progenitor cells in a region A of a culture substrate comprising a region A provided with a surface having cell adhesiveness and a region B provided around the region A and with less cell adhesiveness than the region A, by culturing pluripotent stem cells in an adhesive state in the region A in the presence of a differentiation-inducing factor, wherein the surface of the region A is coated with a cell-adhesive material, and has a contact angle within a predetermined range before being coated with the cell-adhesive material.

When coated with the cell-adhesive material, the contact angle of the surface of the region A is 40 to 80 degrees with a cell-adhesive material solution, or 54 to 77 degrees with water.

### [Culture substrate]

The culture substrate may be made of any material as long as it can have the region A and the region B on its surface. For example, the culture substrate may be made of an inorganic material such as metal, glass, and silicone, or an organic material such as plastic. In some embodiments, the culture substrate is made of glass or plastic, especially polystyrene resin. In some embodiments, the culture substrate is made of glass or plastic, especially polystyrene resin.

The culture substrate is not particularly limited, and may have any shape commonly used for cell culture. The culture substrate may be, for example, a culture vessel such as a petri dish, plate, bottle, chamber, or multi-well plate, or may be a film or porous membrane.

The culture substrate may have a convex portion (also called a pillar) on the surface and the convex portion may have the region A on its upper surface. The shape of the convex portion may be, but is not limited to, a circular cylinder or a prism.

The surface of the culture substrate may be subjected to a known treatment for enhancing cell adhesiveness (such as plasma treatment or corona discharge treatment).

### [Region A]

The region A is a region that can maintain pluripotent stem cells in adhesive culture. The region A is preferably formed by adding dropwise a cell-adhesive material solution onto a partial region of the surface of the culture substrate to coat it with the cell-adhesive material.

When coating with the cell-adhesive material solution, if the contact angle of the surface of the region A is 40 to 80 degrees with the cell-adhesive material solution, or 54 to 77 degrees with water, it is possible to form a neural retinal progenitor cell layer with uniform thickness in the region A. In addition, it is possible to suppress the contamination of the neural retinal progenitor cell layer formed in the region A with ciliary marginal zone-like cells and retinal pigment epithelium-like cells.

The contact angle with the cell-adhesive material solution is preferably 50 degrees to 70 degrees. The contact angle with water is preferably 58 to 72 degrees, and more preferably 62 to 69 degrees.

The contact angle of the cell-adhesive material solution refers to the contact angle at one second after adding dropwise the cell-adhesive material solution onto the surface of the region A. The contact angle of water refers to the contact angle at five seconds after adding dropwise water onto the surface of the region A.

The contact angle was measured in accordance with the sessile drop method as specified in the Japanese Industrial Standard (JIS R 3257:1999), "Testing method of wettability of glass substrate." Specifically, 1 µl of the cell-adhesive material solution or distilled water was left to stand on the surface of the test culture substrate placed horizontally. After a predetermined time (1 second or 5 seconds) had passed, the contact angle was measured using an automated contact angle meter (automated contact angle meter DSA100, KRUSS) for the cell-adhesive material solution, and an automated contact angle meter (automated contact angle meter DSA100S, KRUSS) for distilled water.

In one embodiment, the region A can be formed by adding dropwise the cell-adhesive material solution onto a partial region of the surface of the culture substrate to coat it with the cell-adhesive material. In this case, the region of the surface of the culture substrate that is not coated with the cell-adhesive material solution is defined as the region B.

In another embodiment, the region A can be formed by performing the following steps.

A step 1 of forming a low cell-adhesion layer on a surface of the culture substrate.

A step 2 of removing a portion of the low cell-adhesion layer on the surface of the culture substrate to expose the surface of the culture substrate.

A step 3 of adding dropwise the cell-adhesive material solution onto the exposed region of the surface of the culture substrate to coat it with the cell-adhesive material and form the region A.

In this case, the region of the surface of the culture substrate where the low cell-adhesion layer is maintained in the steps 2 and 3 is defined as the region B. The low cell-adhesion layer can be formed, for example, by coating with the non-cell-adhesive material described below. In addition, the low cell-adhesion layer can be removed, for example, by mechanically scraping it off.

The contact angle of the cell-adhesive material solution may vary within the above numerical range depending on the type and concentration of the cell-adhesive material. For example, when the cell-adhesive material is a human laminin 511-E8 fragment and the concentration of the solution is 5 to 10 ng/µl, the contact angle can be 40 to 80 degrees.

In addition, the contact angle of the cell-adhesive material solution may vary within the above numerical range depending on the type of solvent. For example, when the cell-adhesive material solution is a phosphate buffer solution of human laminin 511-E8 fragment, the contact angle can be 40 to 80 degrees.

The shape of the region A is not limited. In some embodiments, the region A has a circular shape. As used herein, a circular shape refers to a shape that is recognized as substantially circular in the art, including a perfect circle. The area of the region A may be, but is not limited to, 0.5 to 10 cm², 0.7 to 10 cm², 1 to 10 cm², 0.5 to 5 cm², 0.7 to 5 cm², or 1 to 5 cm².

### [Region B]

The region B is a region having cell adhesiveness lower than that of the region A so that, when pluripotent stem cells are seeded on the region A, the cells are prevented from extending from the region A to the region B adjacent to the region A during the induction of retinal tissue differentiation (i.e. the period until differentiation to a retinal tissue is confirmed). The region B may be a region of the surface of the culture substrate that is not coated with the cell-adhesive material solution.

The cell adhesiveness can be compared by seeding pluripotent stem cells of an excess amount relative to an area (e.g. 5×10⁵ to 10×10⁵ cells/cm²) and comparing the percentage of the area to which the cells adhere after 24 hours culture at 37°C and 5% CO₂. The cell adhesiveness is typically evaluated after the medium is removed after the 24 hours culture and the cells are washed with a medium or buffer to remove unadhered cells. The region A may be, for example, a region where, when pluripotent stem cells of an excess amount relative to its area (e.g. 5×10⁵ to 10×10⁵ cells/cm²) are seeded and cultured at 37°C and 5% CO₂ for 24 hours, the cells adhere to 70% or more, 80% or more, or 90% or more of the area after the 24 hours.

In some embodiments, the region B is a region having no cell adhesiveness. The region having no cell adhesiveness refers to a region that does not have sufficient cell adhesiveness to maintain pluripotent stem cells in adhesive culture. The region having no cell adhesiveness may be, for example, a region where, when pluripotent stem cells of an excess amount relative to its area (e.g. 5×10⁵ to 10×10⁵ cells/cm²) are seeded and cultured at 37°C and 5% CO₂ for 24 hours, the cells adhere to 30% or less, 20% or less, or 10% or less of the area after the 24 hours.

The region B may be a region of the exposed surface of a culture substrate having cell adhesiveness lower than that of the region A, or a region of the surface of a culture substrate coated with a material to make the region less cell-adhesive than the region A. In some embodiments, the region B is coated with a non-cell-adhesive material. Examples of non-cell-adhesive materials include MPC (2-methacryloyloxyethyl phosphorylcholine) polymers; celluloses such as methyl cellulose, hydroxyethyl cellulose, hydroxypropyl cellulose, hydroxypropyl methyl cellulose, and sodium carboxymethyl cellulose; polyethylene oxide; carboxyvinyl polymers; polyvinylpyrrolidone; polyethylene glycol; polyamides such as polyacrylamide and poly-N-isopropylacrylamide; polysaccharides such as chitin, chitosan, hyaluronic acid, alginic acid, starch, pectin, carrageenan, guar gum, gum arabic, and dextran; albumin and their derivatives. In some embodiments, the non-cell-adhesive material is an MPC polymer.

The regions A and B may be formed on the surface of a culture substrate by any method used for cell patterning. The regions A and B for example can be formed on the surface of a culture substrate by techniques such as soft lithography, photolithography, and 3D printing.

For example, the regions A and B may be formed on a culture substrate by preparing a sheet with a hole of the shape and size of the region A using a 3D printed mold, covering the surface of the culture substrate with the sheet, coating the portion of the surface not covered with the sheet with a cell-adhesive material, and then removing the sheet.

Alternatively, the regions A and B may be formed on a culture substrate by preparing a sheet of the shape and size of the region A, placing the sheet on the surface of the culture substrate, coating the portion of the surface not covered with the sheet with a non-cell-adhesive material, removing the sheet, and coating the portion of the surface covered with the sheet with a cell-adhesive material. The sheet may be prepared from a biocompatible material such as polydimethylsiloxane (PDMS), polyethylene glycol hydrogel, or agarose gel.

Furthermore, the regions A and B can also be formed on the culture substrate by coating the entire surface of the culture substrate with a non-cell-adhesive material, subsequently removing a portion of the non-cell-adhesive material on the surface of the culture substrate to expose the surface of the culture substrate in a shape and size corresponding to the region A, and then adding dropwise a cell-adhesive material solution onto the exposed surface to coat it with the cell-adhesive material.

The coating can be performed, for example, by bringing the culture substrate into contact with a solution of a cell-adhesive or non-cell-adhesive material and allowing it to react with the solution at 37°C or room temperature for the required time (e.g., 1 hour or more). The concentration of the cell-adhesive or non-cell-adhesive material can be appropriately determined by those skilled in the art, and, for example, for laminin, may be 0.1 to 1 µg/cm², 0.1 to 0.5 µg/cm², or about 0.25 µg/cm².

### [Cell-adhesive material]

The cell-adhesive material is preferably one or more selected from the group consisting of positively charged polymers, laminin, collagens, tenascin, fibrillin, fibronectin, vitronectin, elastin, entactin, proteoglycans, glycosaminoglycans, vitronectin derivatives, and Matrigel.

Examples of positively charged polymers include poly L-lysine and poly L-ornithine.

Examples of collagens include type I collagen, type II collagen, type III collagen, type IV collagen, type V collagen, and type VII collagen.

Examples of proteoglycans include proteoglycans composed of sulfated glycosaminoglycans such as chondroitin sulfate, heparan sulfate, keratan sulfate, and dermatan sulfate and core proteins.

Examples of glycosaminoglycans include chondroitin sulfate, heparan sulfate, keratan sulfate, dermatan sulfate, and hyaluronic acid.

The cell-adhesive material is more preferably laminin. Laminin is a heterotrimeric molecule comprising three subunit chains, α, β, and γ. There are five types of α chains, α1 to α5, three types of β chains, β1 to β3, and three types of γ chains, γ1 to γ3, and each laminin isoform is represented by the numbers indicating its constituent subunits (for example, laminin 111 is composed of α1 chain, β1 chain, and γ1 chain). As the laminin, laminin 111, laminin 121, laminin 211, laminin 213, laminin 222, laminin 311 (laminin 3A11), laminin 332 (laminin 3A32), laminin 321 (laminin 3A21), laminin 3B32, laminin 411, laminin 421, laminin 423, laminin 521, laminin 522, laminin 523, or fragments thereof (for example, laminin 211-E8, laminin 311-E8, laminin 411-E8, and laminin 511-E8, which are E8 fragments) can be used.

The cell-adhesive material is even more preferably a human laminin 511-E8 fragment.

### [Induction of differentiation]

In the method for producing a tissue comprising neural retinal progenitor cells according to the present disclosure, pluripotent stem cells are seeded onto the region A, and adhesive culture is performed by the following steps.

Step 1: a step of culturing pluripotent stem cells in a medium containing a BMP signaling inhibitor, as well as serum and/or a serum substitute.

Step 2: a step, following the step 1, of culturing the cells obtained in the step 1 in a medium containing a BMP signaling activator, as well as serum and/or a serum substitute.

Step 3: a step, following the step 2, of culturing the cells obtained in the step 2 in a medium free of both BMP signaling inhibitor and BMP signaling activator, and containing serum and optionally a serum substitute.

No additional steps are included between the step 2 and the step 3, and particularly, no step of culturing the cells in the absence of serum. By subjecting the neural retinal progenitor cells differentiated in the step 2 to culture in the medium containing serum of the step 3 without going through a culture in a serum-free medium, it is possible to suppress cell death of the neural retinal progenitor cells and to form a neural retinal progenitor cell layer with uniform thickness in the region A. The medium used in the step 3 may contain serum, or may contain a serum substitute in addition to the serum.

The pluripotent stem cells are seeded on a culture substrate at a cell number so that the cells occupy 70% or more of the region A. The cells may be seeded only in the region A or on the entire region of the culture substrate including the region A and the region B. For example, when the region B is a non-cell-adhesive region, the cells can be seeded on the entire region of the culture substrate. For example, the pluripotent stem cells may be seeded at 5×10⁵ to 10x10⁵ cells/cm². Typically, pluripotent stem cells are collected and dispersed with a cell dispersing solution containing an enzyme (e.g., trypsin, collagenase, hyaluronidase, elastase, pronase, DNase, or papain) and/or a chelating agent (e.g., ethylenediaminetetraacetic acid (EDTA)), and suspended in a desired medium. Then, the resulting cell suspension is added onto the culture substrate. As the cell dispersing solution, for example, a commercial product such as TrypLE^{™} Select (Thermo Fisher Scientific) or TrypLE^{™} Express (Thermo Fisher Scientific) may be used.

Differentiation induction of pluripotent stem cells into a retinal tissue is performed by adhesive culture on the culture substrate. The differentiation induction of pluripotent stem cells into a retinal tissue may be started after pluripotent stem cells seeded on a culture substrate are cultured for a certain period of time in a medium containing a factor for maintaining undifferentiated state. In the present disclosure, the time when the culturing in a medium that does not contain a factor for maintaining undifferentiated state is started is defined as the time when the differentiation induction into a retinal tissue is started.

The medium can be prepared by using a medium commonly used for culturing animal cells as a basal medium. Examples of basal media include IMDM, DMEM, F-12, DMEM/F12, IMDM/F12, BME, BGJb, CMRL 1066, Glasgow MEM (GMEM), Improved MEM Zinc Option, Medium 199, Eagle MEM, alpha MEM, Ham's medium, RPMI 1640, Fischer's medium, and mixtures of thereof.

To the basal medium, one or more components selected from serum, serum substitutes, growth factors, factors for maintaining undifferentiated state, cytokines, insulin, fatty acids, lipids, vitamins, amino acids (e.g., nonessential amino acids, retinoids, glutamine, taurine), antioxidants, 2-mercaptoethanol, 1-thioglycerol, antibiotics, buffers, and inorganic salts can be added as needed.

Specific examples of serum substitutes include KnockOut^{™} Serum Replacement (Thermo Fisher Scientific), Chemically Defined Lipid Concentrate (Thermo Fisher Scientific), GlutaMAX^{™} Supplement (Thermo Fisher Scientific), Ham's F-12 Nutrient Mix, GlutaMAX^{™}Supplement (Thermo Fisher Scientific), B27 Supplement (Thermo Fisher Scientific), N2 Supplement (Thermo Fisher Scientific), and ITS Supplement (Thermo Fisher Scientific).

The medium may be a commercially available medium that contains any of the above components in a basal medium, such as Ham's F-12 Nutrient Mix, GlutaMAX^{™}Supplement (Thermo Fisher Scientific) or DMEM/F-12, GlutaMAX^{™}Supplement (Thermo Fisher Scientific).

In some embodiments, the BMP signaling inhibitor of the step 1 is LDN-193189. The concentration of a BMP signaling inhibitor is determined according to the inhibitor to be used, and may be 1 to 1000 nM, 10 to 500 nM, 30 to 300 nM, or about 100 nM.

The period of culturing of the step 1 may be, but is not limited to, 1 to 10 days, 2 to 9 days, 3 to 7 days, 4 to 6 days, or about 5 days.

In some embodiments, the signaling activator of the step 2 is BMP4. The concentration of a BMP signaling activator is determined according to the activator to be used, and may be 0.01 to 1000 nM, 0.1 to 100 nM, 1 to 10 nM, 1 to 3 nM, or about 1.5 nM.

The culturing of the step 2 is performed for a period necessary for differentiation into a retinal tissue. The period of culturing may be, but is not limited to, 1 to 30 days, 2 to 20 days, 3 to 15 days, 4 to 12 days, or 5 to 10 days (e.g., 5, 6, 7, 8, 9, or 10 days).

The concentration of a BMP signaling activator may be constant or varied during the culturing period. For example, the concentration of a BMP signaling activator may be decreased stepwise at a rate of 40 to 60% reduction per 2 to 4 days. For example, the concentration of a BMP signaling activator in a medium may be decreased stepwise by replacing a portion (e.g., half) of the medium containing the BMP signaling activator with a medium not containing the BMP signaling activator every 2, 3, or 4 days after the start of culturing in the medium containing the BMP signaling activator.

In some embodiments, the medium containing a BMP signaling inhibitor or BMP signaling activator is prepared from a 1:1 mixture of F-12 medium and IMDM medium supplemented with KnockOut^{™} Serum Replacement (Thermo Fisher Scientific) (e.g., 0.5% to 30%, 1% to 20%, or 10%), Chemically Defined Lipid Concentrate (Thermo Fisher Scientific), BSA, and 1-thioglycerol.

The period of culturing of the step 3 may be, but is not limited to, 1 to 100 days, 10 to 90 days, 20 to 80 days, 30 to 70 days, 40 to 60 days, or about 50 days.

During the culturing period, the medium may be replaced as needed. For example, a part or the whole of the medium may be replaced every 1 to 4 days.

Culture conditions such as culture temperature and CO₂ concentration can be appropriately determined. The culture temperature may be, for example, 30 to 40°C or about 37°C. The CO₂ concentration may be, for example, 1% to 10% or about 5%.

Differentiation into a retinal tissue can be confirmed by detecting expression of a cell marker in cells of the tissue. The expression of a marker may be confirmed, for example, on any of Days 10 to 100 of differentiation induction (e.g., Day 10, 11, 12, 13, 14, 15, 16, 17, 18, 19, 20, 21, 22, 23, 24, 25, 26, 27, 28, 29, 30, 40, 50, 60, 70, 80, 90, or 100), or later. In some embodiments, the expression of a marker is confirmed on any of Days 18 to 22 of differentiation induction.

### 2. Method for producing neural retinal sheet for transplantation and composite sheet for retinal transplantation

The method for producing a neural retinal sheet for transplantation according to the present disclosure includes a step of producing a tissue (retinal organoid) comprising neural retinal progenitor cells by the production method described above, and a step of excising a neural retinal progenitor cell layer from the tissue to obtain the neural retinal sheet for transplantation.

In addition, the method for producing a composite sheet for retinal transplantation according to the present disclosure includes a step of laminating this neural retinal sheet for transplantation onto a retinal pigment epithelial cell sheet using a flowable gel to produce the composite sheet for retinal transplantation.

According to the method for producing a tissue (retinal organoid) comprising neural retinal progenitor cells of the present disclosure, it is possible to form a neural retinal progenitor cell layer with uniform thickness in the region A, which allows excision of a relatively large cell layer with a thickness suitable for transplantation when excising the neural retinal progenitor cell layer from the tissue (retinal organoid), thus improving the production efficiency of the neural retinal sheet for transplantation, or the composite sheet for retinal transplantation. In addition, according to the method for producing a tissue (retinal organoid) comprising neural retinal progenitor cells of the present disclosure, it is possible to suppress the contamination of the neural retinal progenitor cell layer formed in the region A with ciliary marginal zone-like cells and retinal pigment epithelium-like cells, which allows excision of a neural retinal progenitor cell layer that is less contaminated with ciliary marginal zone-like cells and retinal pigment epithelium-like cells, thus improving the quality of the neural retinal sheet for transplantation, or the composite sheet for retinal transplantation.

The retinal organoid preferably has a diameter of about 1 cm or more so that a plurality of neural retinal sheets for transplantation can be excised therefrom. Herein, a diameter of 1 cm or more refers to the length of the portion corresponding to the diameter in the case where the retinal organoid has a shape close to a circle, or to the major axis in the case where the retinal organoid has a shape close to an ellipse.

In the present disclosure, "about" indicates a value that varies up to ±10%, ±8%, ±6%, ±5%, ±4%, ±3%, ±2%, or ±1% relative to the reference value. Preferably, the term "about" indicates a range of ±10%, ±5%, or ±1% relative to the reference value.

The excision of neural retinal sheets for transplantation from the retinal organoid can be performed by a known method. For example, a grid corresponding to the size of the sheet is set on the retinal organoid, and aligned such that the number of sheets obtainable from the retinal organoid (effective number) is maximized, then the fragments are excised along the grid.

The excised neural retinal sheet for transplantation preferably has a size that can be loaded into a cannula for transplantation, with a length of 1.0 to 10.0 mm in the long axis direction and a length of 0.5 to 2.0 mm in the short axis direction. For example, when using a 24G cannula, the length in the minor axis direction is about 0.5 to about 1.0 mm, while the length in the major axis direction can be freely set. When using a 18G cannula, the length in the minor axis direction can also be extended up to about 2.0 mm. By filling the cannula with a low-viscosity viscoelastic substance, such as a 7-fold diluted hyaluronic acid for ophthalmic surgery (e.g., Viscoat), it is possible to load a plurality of sheets. Particularly, when using a 24G cannula, it is possible to transplant the fragments to the intended area with a small volume of medium, within about 10 µl, without making a large incision in the retina.

For the preparation of the neural retinal sheet for transplantation, it is preferable to use a planar retinal organoid that is about 40 to 90 days, more preferably about 45 to 80 days, and particularly preferably about 50 to 70 days, from the start of differentiation of pluripotent stem cells. This is because sufficient retinal differentiation from pluripotent stem cells can be expected for a retinal organoid at this stage. A planar retinal organoid at about 60 days of differentiation is characterized by a bilayer structure containing primarily more immature retinal progenitor cells (VSX2- or RX-positive) on the parietal side, and more differentiated inner retinal cells such as ganglion cells (BRN3B, PAX6, ISL1) on the basal membrane side.

The neural retinal sheet for transplantation is composed of a retinal organoid in which the ratio of neural retinal progenitor cells to all cells is about 90% or more, and more preferably about 95% or more, and the cells are forming a layered structure.

The retinal sheet for transplantation is characterized by having a high expression of one or more selected from the photoreceptor progenitor cell markers CRX, Recoverin, and Rxr-gamma, and low expression or below the detection limit of one or more selected from the extraretinal tissue cell markers CRYAB, ZIC1, and EMX2.

The neural retinal sheet for transplantation may be laminated with retinal pigment epithelial cells to form a composite sheet. *In vivo,* the retina and retinal pigment epithelial cells are in contact with each other on their parietal surfaces, and do not require strong adhesion. Therefore, a low-viscosity flowable gel, such as x7 Viscoat (mixture of sodium chondroitin sulfate and sodium hyaluronate for ophthalmic surgery), can be used to loosely adhere the retinal sheet for transplantation and the retinal pigment epithelial cell sheet, and obtain a composite sheet that can be used for transplantation.

As a flowable gel, a histocompatible flowable gel can be used, including polysaccharides such as hyaluronic acid and chondroitin sulfate that are present *in vivo* as an extracellular matrix between the retina and the retinal pigment epithelial cells. These flowable gels can also be used as the transplantation medium during sheet insertion, as well as components of the preservation solution for the sheets.

### 3. Composition and cell-based medicament for treating retinal diseases

The present disclosure also provides a tissue comprising neural retinal progenitor cells, obtained by the method for producing a tissue comprising neural retinal progenitor cells described above, and a composition or cell-based medicament for treating retinal diseases comprising the tissue.

The composition or cell-based medicament for treating retinal diseases may be the neural retinal sheet for transplantation or composite sheet for retinal transplantation described above. The cell-based medicament may contain a transplantation medium that also functions as a preservation solution for the neural retinal sheet for transplantation or composite sheet for retinal transplantation.

The composition or cell-based medicament for treating retinal diseases may comprise a pharmaceutically acceptable carrier in addition to the tissue comprising neural retinal progenitor cells. Examples of the pharmaceutically acceptable carrier include a physiological aqueous solvent such as saline, buffer solution, or serum-free medium. The composition may further comprise an additive such as a preservative, stabilizer, reducing agent, and isotonic agent. The composition or cell-based medicament for treating retinal diseases may be cryopreserved, and in this case, the composition or the like may comprise a suitable cryoprotectant.

The tissue comprising neural retinal progenitor cells may also be used for evaluating toxicity or efficacy of an agent on retinal tissues. In some embodiments, the tissue of the present disclosure is used for evaluating toxicity or efficacy of a therapeutic agent for a retinal disease.

### 4. Method for treating retinal diseases

The present disclosure also provides a method for treating retinal diseases, comprising a procedure of transplanting the composition or cell-based medicament for treating retinal diseases, the neural retinal sheet for transplantation, or the composite sheet for retinal transplantation described above into the retina of a subject.

Examples of retinal diseases include retinitis pigmentosa, age-related macular degeneration, diabetic retinopathy, retinopathy of newborn babies, macular dystrophy, cone-rod dystrophy, rod-cone dystrophy, macular hole, chloroquine retinopathy, degenerative myopia, traumatic macular disease, glaucoma, and organic mercury poisoning, which are diseases in which visual function is impaired due to damage to cells constituting the retinal tissue.

The neural retinal sheet for transplantation and the composite sheet for retinal transplantation can be transplanted into the retina of a subject in need thereof, for example, by loading them into a 24G cannula.

### Examples

### [Reference Example 1: Production of sheet-like retinal tissue from pluripotent stem cells]

A sheet-like retinal tissue (hereinafter also referred to as "planar retinal organoid") was produced according to the method described in Patent Literature 1. The overview of the procedure is shown below.

### Day 0

A circular PMDS sheet having a circular hole with a diameter of 1 cm was placed on a dish (the PMDS sheet and the dish had the same diameter). The hole of the PMDS sheet was filled with 200 µl of iMatrix-511 solution (7 ng/µl phosphate buffer solution of human laminin 511-E8 fragment), followed by coating in a 37°C incubator for 1 hour to form the region A (37°C for 1 hour).

After removing the iMatrix-511 solution with an aspirator, 5×10⁵ iPS cells are suspended in 200 µl of StemFIT^{®} AK03N medium supplemented with Y27632 at a final concentration of 10 µM, and seeded onto the region A. After allowing the cells to stand in a 37°C incubator for 30 minutes, the PMDS sheet is removed using sterile tweezers (region B), and 300 µl of the same medium is added, followed by culture in a 5% CO₂ incubator.

### Day 1

The medium is replaced with 500 µl of StemFIT AK03N (without Y27632 and supplemented with penicillin/streptomycin).

### Day 2

After washing the cells three times with 1 ml of gfCDM (10% KSR), the medium is replaced with 1 ml of gfCDM (10% KSR).

### [Composition of gfCDM (10% KSR) medium]

| | |
|---|---|
| IMDM (31980-030) | 125 ml |
| F-12 (31765-092) | 125 ml |
| CD lipid (11950-031) | 2.5 ml |
| BSA (5g/20ml) | 5 ml |
| 1-Thioglycerol (207-09232) | 9.75 µl |
| Penicillin/streptomycin | 2.5 ml |
| KSR | 25 ml |

### Day 3 and Day 6

The medium is replaced with 1 ml of gfCDM medium (10% KSR) containing 100 nM LDN-193189.

### Day 8

After washing the cells with 1 ml of gfCDM (10% KSR), the medium is replaced with 1 ml of gfCDM (10% KSR) containing BMP4 (final concentration of 1.5 nM).

### Day 11 and Day 14

Half of the medium is replaced with BMP4-free gfCDM (10% KSR).

### Day 17

After washing the cells three times with DMEM/F-12 + Glutamax, the medium is replaced with 1 ml of the medium described below.

### [Medium Composition]

| | |
|---|---|
| DMEM/F-12+Glutamax (10565-042) | 5 ml |
| N2 supplement (17502-048) | 50 µl |
| Penicillin/streptomycin | 50 µl |

### Day 20

The medium is replaced with 1 ml of the following control mature medium.

### [Medium Composition]

| | |
|---|---|
| DMEM/F-12+Glutamax (10565-042) | 500 ml |
| N2 supplement (17502-048) | 5 ml |
| Inactivated FBS | 50 ml |
| 50 mM Taurine (in PBS) | 1.12 ml |
| Antibiotic-Antimycotic (100x) (15240-062) | 5 ml |

| | |
|---|---|
| *Thereafter, the medium is replaced once every 2 to 3 days. | |

### [Test Example 1: Study of contact angle of surface of region A]

A planar retinal organoid was produced by modifying the procedure on "Day 0" of Reference Example 1 as follows. Except for the procedure on "Day 0", the procedure was the same as in Reference Example 1.

80 µl of iMatrix-511 solution was added dropwise onto the center of the dish and allowed to settle for about one minute. Then, 30 µl of iMatrix-511 solution was collected, and 50 µl of circular droplet of iMatrix-511 solution was retained while being coated in a 37°C incubator for 1 hour to form the region A.

After removing the iMatrix-511 solution with an aspirator, iPS cells were seeded onto the region A and cultured in the same manner as in Reference Example 1.

For the dish, a dish A having a contact angle of 58.5 degrees one second after adding dropwise 1 µl of iMatrix-511 solution onto its surface, and a dish B having a contact angle of 30.0 degrees, were used. In the Examples, the contact angle was measured at five independent points on the surface of the dish, and the average of these measurements was used as the contact angle of the dish.

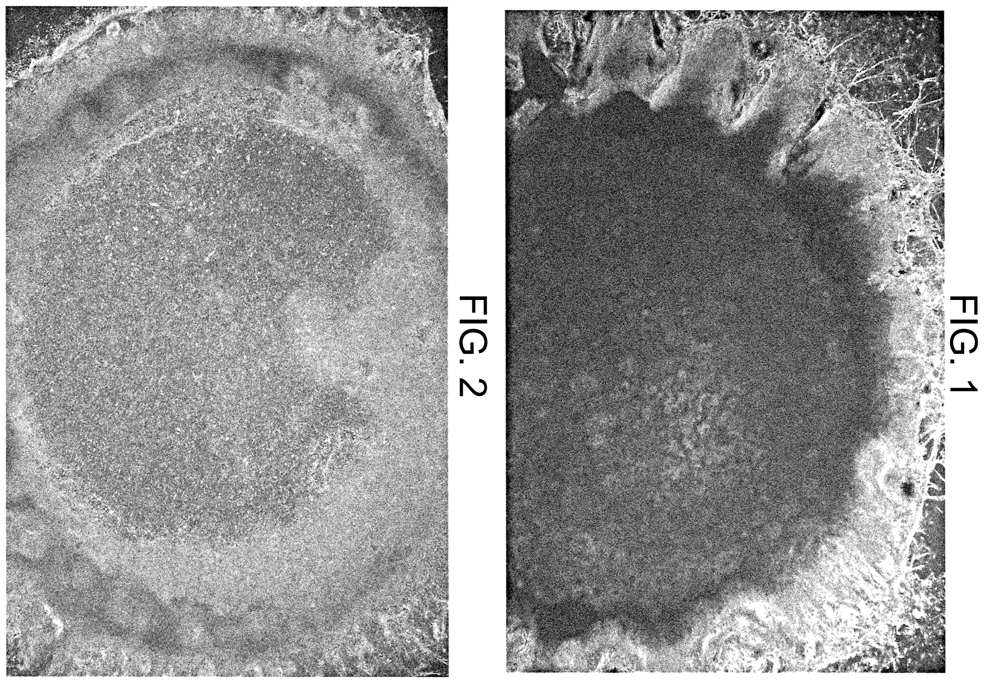

Phase-contrast microscopic images on Day 37 of the planar retinal organoid are shown in Figures 1 and 2. In the dish A, the region appearing dark in the image (corresponding to the region A) indicates uniform stratification of the neural retinal progenitor cell layer (see Figure 1). On the other hand, in the dish B, many regions in the image appeared bright, and the formation of the neural retinal progenitor cell layer was uneven (see Figure 2).

It was shown that by using a culture substrate in which the contact angle of the cell-adhesive material solution on the substrate surface is about 58 degrees, a neural retinal progenitor cell layer having uniform thickness can be formed.

Furthermore, a test was conducted in the same manner using dishes C to J, having contact angles of 24 degrees, 55 degrees, 56 degrees, 60 degrees, 66 degrees, 70 degrees, 76 degrees, and 90 degrees, respectively, five seconds after adding water dropwise onto their surface.

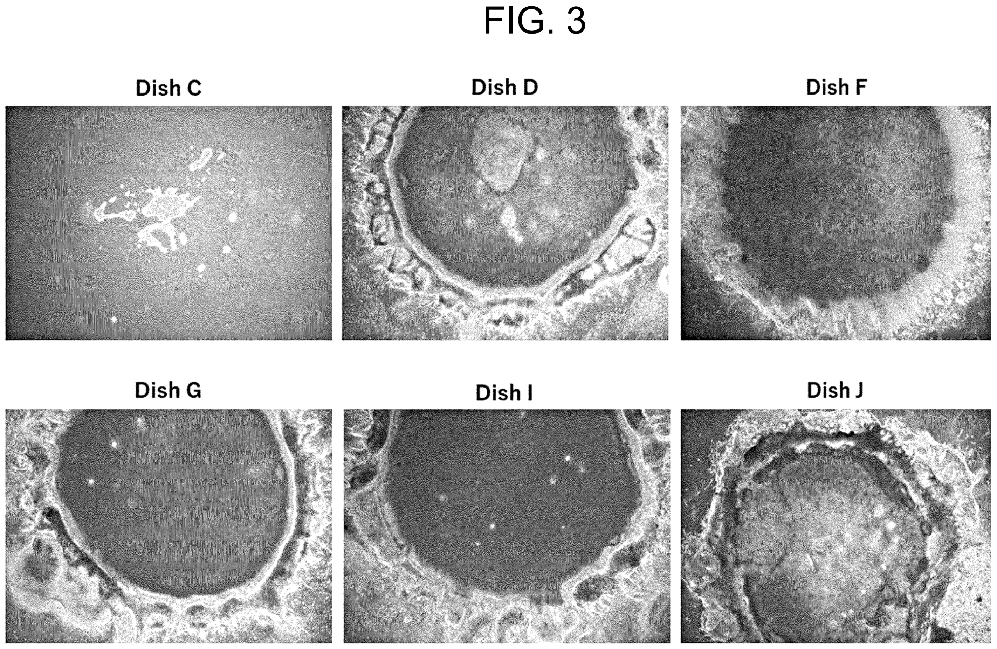

Phase-contrast microscopic images on Day 38 of the planar retinal organoid are shown in Figure 3. In the dish C (water contact angle of 24 degrees), the cells did not adhere. In the dish J (water contact angle of 90 degrees), the formation of the neural retinal progenitor cell layer was uneven.

On the other hand, in the dishes D to I with water contact angles of 55 degrees to 76 degrees, uniform stratification of the neural retinal progenitor cell layer was observed, with particularly good results in dishes E to I, which had water contact angles of 56 degrees to 76 degrees.

### [Test Example 2: Study of medium for maturation of neural retinal progenitor cells]

A planar retinal organoid was produced by modifying the procedure on "Day 17" of Reference Example 1 as follows. Except for the procedure on "Day 17", the procedure was the same as in Reference Example 1.

After washing three times with DMEM/F-12 + GlutaMAX, the medium is replaced with 1 ml of the medium described below.

### [Medium Composition]

| | |
|---|---|
| DMEM/F-12+Glutamax (10565-042) | 500 ml |
| N2 supplement (17502-048) | 5 ml |
| Inactivated FBS | 50 ml |
| 50 mM Taurine (in PBS) | 1.12 ml |
| Antibiotic-Antimycotic (100x) (15240-062) | 5 ml |

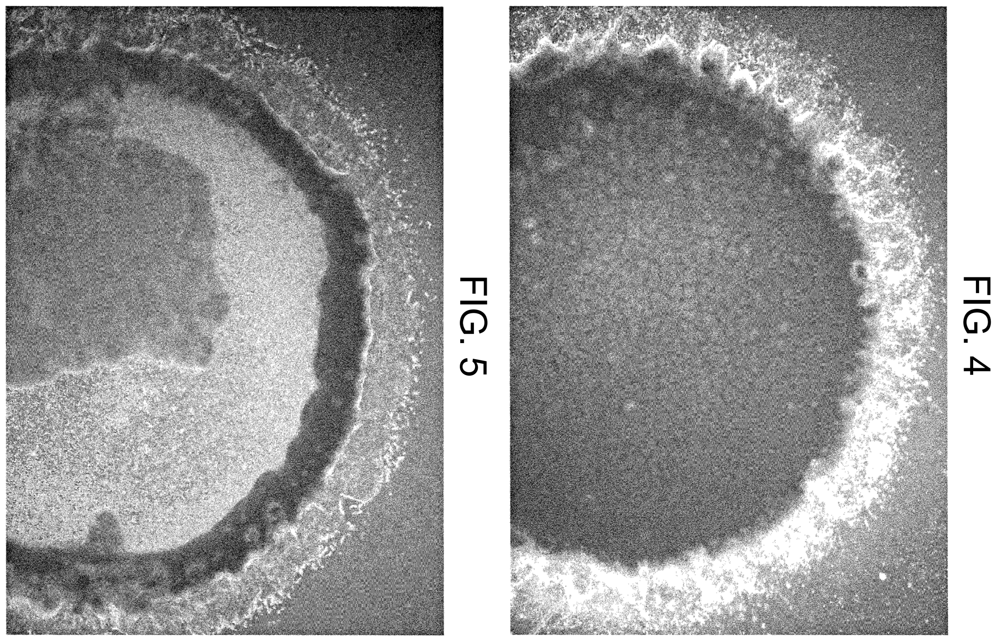

Phase-contrast microscopic images on Day 20 of the planar retinal organoid are shown in Figures 4 and 5. In the method of Reference Example 1, the medium was replaced with a medium free of serum on "Day 17", and from "Day 20" onwards, the medium was replaced with a medium containing serum before performing the culture. However, with this method, loss of the neural retinal progenitor cell layer (regions appearing bright in the image) was sometimes observed on "Day 20" (see Figure 5). This is thought to be due to the cell death of the neural retinal progenitor cells. On the other hand, when culturing in a medium containing serum from "Day 17", no loss of neural retinal progenitor cells was observed on "Day 20" (see Figure 4).

It was shown that, by subjecting the neural retinal progenitor cells differentiated by BMP4 treatment to culture in a serum medium without going through a culture in a serum-free medium, it is possible to suppress cell death of the neural retinal progenitor cells and to form a neural retinal progenitor cell layer with uniform thickness.

## Claims

1. A method for producing a tissue comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor,
wherein the surface of the region A is coated with a cell-adhesive material, and has a contact angle within a predetermined range before being coated with the cell-adhesive material.

2. The production method according to claim 1, wherein the surface of the region A has a contact angle of 40 to 80 degrees with a cell-adhesive material solution when coated with the cell-adhesive material.

3. The production method according to claim 1 or 2, wherein the surface of the region A has a contact angle of 54 to 77 degrees with water when coated with the cell-adhesive material.

4. The production method according to any one of claims 1 to 3, further comprising a step of adding dropwise the cell-adhesive material solution onto a partial region of the surface of the culture substrate to coat it with the cell-adhesive material and form the region A, and defining, as the region B, a region of the surface of the culture substrate that is not coated with the cell-adhesive material solution.

5. The production method according to any one of claims 1 to 3, further comprising:
a step of forming a low cell-adhesion layer on a surface of the culture substrate;
a step of removing a portion of the low cell-adhesion layer on the surface of the culture substrate to expose the surface of the culture substrate; and
a step of adding dropwise the cell-adhesive material solution onto the exposed region of the surface of the culture substrate to coat it with the cell-adhesive material and form the region A, and defining, as the region B, a region of the surface of the culture substrate where the low cell-adhesion layer has been maintained.

6. The production method according to any one of claims 1 to 5, wherein the cell-adhesive material is one or more selected from the group consisting of positively charged polymers, laminin, collagens, tenascin, fibrillin, fibronectin, vitronectin, elastin, entactin, proteoglycans, glycosaminoglycans, vitronectin derivatives, and Matrigel.

7. The production method according to claim 6, wherein the cell-adhesive material is laminin.

8. The production method according to any one of claims 1 to 7, wherein the culture in an adhesive state comprises:
a step 1 of culturing the pluripotent stem cells in a medium comprising a BMP signaling inhibitor, as well as serum and/or a serum substitute;
a step 2, following the step 1, of culturing the cells obtained in the step 1 in a medium comprising a BMP signaling activator, as well as serum and/or a serum substitute; and
a step 3, following the step 2, of culturing the cells obtained in the step 2 in a medium free of both BMP signaling inhibitor and BMP signaling activator, and comprising serum and optionally a serum substitute.

9. A method for producing a tissue comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor,
wherein the culture in an adhesive state comprises:
a step 1 of culturing the pluripotent stem cells in a medium comprising a BMP signaling inhibitor, as well as serum and/or a serum substitute;
a step 2, following the step 1, of culturing the cells obtained in the step 1 in a medium comprising a BMP signaling activator, as well as serum and/or a serum substitute; and
a step 3, following the step 2, of culturing the cells obtained in the step 2 in a medium free of both BMP signaling inhibitor and BMP signaling activator, and comprising serum and optionally a serum substitute.

10. The production method according to claim 9, wherein the surface of the region A is coated with a cell-adhesive material, and has a contact angle within a predetermined range before being coated with the cell-adhesive material.

11. The production method according to claim 10, wherein the surface of the region A has a contact angle of 40 to 80 degrees with a cell-adhesive material solution when coated with the cell-adhesive material.

12. The production method according to claim 10 or 11, wherein the surface of the region A has a contact angle of 54 to 77 degrees with water when coated with the cell-adhesive material.

13. The production method according to any one of claims 10 to 12, further comprising a step of adding dropwise the cell-adhesive material solution onto a partial region of the surface of the culture substrate to coat it with the cell-adhesive material and form the region A, and defining, as the region B, a region of the surface of the culture substrate that is not coated with the cell-adhesive material solution.

14. The production method according to any one of claims 10 to 12, further comprising:
a step of forming a low cell-adhesion layer on a surface of the culture substrate;
a step of removing a portion of the low cell-adhesion layer on the surface of the culture substrate to expose the surface of the culture substrate; and
a step of adding dropwise the cell-adhesive material solution onto the exposed region of the surface of the culture substrate to coat it with the cell-adhesive material and form the region A, and defining, as the region B, a region of the surface of the culture substrate where the low cell-adhesion layer has been maintained.

15. The production method according to any one of claims 10 to 14, wherein the cell-adhesive material is one or more selected from the group consisting of positively charged polymers, laminin, collagens, tenascin, fibrillin, fibronectin, vitronectin, elastin, entactin, proteoglycans, glycosaminoglycans, vitronectin derivatives, and Matrigel.

16. The production method according to claim 15, wherein the cell-adhesive material is laminin.

17. A tissue comprising neural retinal progenitor cells, obtained by the production method according to claim 1 or 9.

18. A composition for treating retinal diseases, comprising the tissue according to claim 17.

19. A method for producing a neural retinal sheet for transplantation from a retinal organoid derived from pluripotent stem cells, the method comprising:
a step of producing a retinal organoid comprising neural retinal progenitor cells in a region A of a culture substrate comprising a region A provided with a surface having cell adhesiveness and a region B provided around the region A and with a surface having less cell adhesiveness than the region A, by culturing pluripotent stem cells in an adhesive state in the region A in the presence of a differentiation-inducing factor, wherein the surface of the region A is coated with a cell-adhesive material, and has a contact angle within a predetermined range before being coated with the cell-adhesive material, and
a step of excising a neural retinal progenitor cell layer from the retinal organoid to obtain the neural retinal sheet for transplantation.
